Europäisches Patentamt

European Patent Office

Office européen des brevets

(11) Numéro de publication : **0 338 931 B1**

(12) **FASCICULE DE BREVET EUROPEEN**

(45) Date de publication du fascicule du brevet :
16.09.92 Bulletin 92/38

(51) Int. Cl.⁵ : **A61K 7/02,** A61K 7/48,
A23D 7/00, A23D 9/00

(21) Numéro de dépôt : **89401103.0**

(22) Date de dépôt : **20.04.89**

(54) **Produits nouveaux comportant une émulsion d'eau et d'hydrocarbures paraffiniques huileux additionnés d'extraits de léchithine et procédés de fabrication.**

(30) Priorité : **22.04.88 FR 8805565**

(43) Date de publication de la demande :
**25.10.89 Bulletin 89/43**

(45) Mention de la délivrance du brevet :
**16.09.92 Bulletin 92/38**

(84) Etats contractants désignés :
**AT BE CH DE ES GB GR IT LI LU NL SE**

(56) Documents cités :
**Patent Abstracts of Japan, vol. 10, no. 362
(C-389)(2419), 4 décembre 1986
Patent Abstracts of Japan, vol. 12, no. 22
(C-470)(2869), 22 janvier 1988
Patent Abstracts of Japan, vol. 12, no. 213
(C-505)(3060), 17 juin 1988**

(73) Titulaire : **Dubois, Jacques
8, Boulevard Gambetta
F-11100 Narbonne (FR)**

(72) Inventeur : **Dubois, Jacques
8, Boulevard Gambetta
F-11100 Narbonne (FR)**

(74) Mandataire : **Morelle, Guy Georges Alain
CABINET MORELLE & BARDOU, SC 5,
Boulevard de la Méditerranée
F-31400 Toulouse (FR)**

EP 0 338 931 B1

# Description

La présente invention a pour objet des produits nouveaux notamment des produits diététiques, laxatifs ou cosmétiques, comportant une émulsion d'eau et d'hydrocarbures paraffiniques huileux ou solides à la température ambiante, additionnés d'extraits de lécithine et des procédés de fabrication de ces produits.

Le secteur technique de l'invention est celui de l'élaboration de produits diététiques, laxatifs ou cosmétiques à base d'hydrocarbures paraffiniques et d'extraits de lécithine.

Dans le domaine alimentaire ou diététique, on connaît de nombreux produits pâteux, qui peuvent être étalés sur des tartines par exemple le beurre, les pâtés, les fromages fondus, les crèmes de poissons etc...

L'aptitude de ces produits à être tartinés est due à leur teneur élevée en lipides, qui peut varier de 82 % pour le beurre à 40 % environ pour les beurres dits allégés dans lesquels une partie du beurre est remplacée par des huiles végétales.

Un des problèmes nutritionnels actuels est la trop grande richesse des aliments en lipides qui entraînent des maladies cardio-vasculaires et provoquent l'obésité.

Un premier objectif de la présente invention est de procurer des produits diététiques pâteux tartinables dans lesquels la totalité ou une grande partie des lipides assimilables par l'organisme sont remplacés par des hydrocarbures paraffiniques huileux, non assimilables, ce qui permet d'obtenir des produits qui sont tartinables malgré l'absence ou la faible quantité de lipides et qui ont une valeur énergétique nulle ou très faible.

On connaît déjà des produits alimentaires diététiques ou de régime, dans la composition desquels une partie ou la totalité des matières grasses est remplacée par des huiles minérales.

Le brevet FR.71.46658 (Publication 2.165.312) de M. Kremer, décrit des produits alimentaires à faible pouvoir calorifique et, plus particulièrement, des pâtisseries, des pâtés, du chocolat et des pralinés contenant de l'huile de paraffine ou de vaseline et un agent émulsionnant naturel qui peut être de la lécithine.

Le brevet FR. 652.995 (Becundia Holding) décrit un procédé de fabrication du pain, dans lequel on ajoute dans la pâte une émulsion d'huile de paraffine, de glycérine et d'eau.

Le brevet FR. 914.504 (N.S.B. Patents) décrit un procédé de fabrication de pain, de pâtisserie ou de produits similaires, dans lequel on ajoute dans la farine une faible quantité de cire de paraffine et d'hydrocarbures liquides avec ou sans addition de petites quantités de glycérine ou de lécithine.

Le brevet FR. 933.429 (N.S.B Patents) décrit un procédé dans lequel on ajoute à la pâte une émulsion aqueuse contenant une cire minérale, végétale ou animale et un émulsifiant qui peut être de la lécithine.

Aucun de ces brevets antérieurs ne décrit des produits diététiques à l'état de pâte tartinable composés d'une émulsion d'une phase aqueuse et d'une phase huileuse contenant respectivement un extrait de lécithine hydro-dispersible et un extrait de lécithine liposoluble.

Les trois derniers brevets cités concernent exclusivement des procédés de fabrication de pain ou de pâtisseries à base de farine destinés à améliorer la conservation du pain.

Le brevet FR. 71 .46658 concerne la fabrication de produits alimentaires tels que des pâtisseries cuites à base de farine et d'oeufs, des chocolats, des pralinés ou des pâtés cuits à base de viande et de foie, qui sont des produits alimentaires très différents des produits pâteux selon la présente invention, destinés à servir de substitut au beurre ou à la margarine ou à préparer des pâtes tartinables.

On sait que l'huile de paraffine favorise le transit intestinal et a un effet laxatif.

Toutefois la consistance huileuse a un effet répulsif et de nombreuses personnes répugnent à avaler des laxatifs huileux. Un deuxième objectif de la présente invention est de procurer des pâtes laxatives tartinables faciles à absorber.

On connaît de nombreux produits cosmétiques destinés aux soins cutanés tels que crèmes de jour ou de nuit, des laits de toilette, des crèmes solaires etc...

La plupart des produits cosmétiques connus à ce jour, contiennent dans leur formulation des agents surfactants ou tensio-actifs de synthèse, qui peuvent être des agents anioniques (lauryl sulfate ou lauryl ether sulfate) ou cationique (ammonium quaternaire) non ioniques ou amphotères.

Or les agents surfactants de synthèse ont un caractère agressif pour les cellules vivantes. Lorsqu'ils sont ajoutés, même à très faible concentration, à des cultures cellulaires in vitro, on observe une diminution notable de la multiplication des cellules et une altération de la morphologie de celles-ci.

Les produits dermo-cosmétiques sont généralement appliqués sur la peau pendant des durées relativement longues, de l'ordre de 8 heures à 10 heures et ces applications sont répétées.

La présence d'agents tensio-actifs de synthèse cyto-toxiques dans leur composition entraîne des réactions cutanées tel les que : rougeurs, irritations, démangeaisons.

Chez les personnes à peau sensible ou chez les personnes âgées, ou chez les bébés, ces réactions peuvent entraîner des troubles cutanés plus graves tels que dermatites, allergies etc...

Un troisième objectif de la présente invention est de procurer des produits cosmétiques, notamment des produits destinés aux soins cutanés, dans lesquels les agents tensio-actifs de synthèse sont remplacés par des agents émulsionnants naturels utilisés en association avec des hydrocarbures paraffiniques, qui ne présentent aucune agressivité pour les cellules vivantes.

Les objectifs de l'invention sont atteints au moyen d'une famille de nouveaux produits qui sont des émulsions d'une phase aqueuse dans une phase huileuse.

On connaît par ailleurs par le JP-A- 61-157340, un procédé de préparation d'une émulsion du type huile dans l'eau dans lequel une solution huileuse contenant de la lécithine est mélangée à une phase aqueuse contenant de la caséine ou du beurre pour obtenir une émulsion riche en lipides et protéines.

La phase aqueuse, selon l'invention, contient un extrait de lécithine hydrodispersible, en proportion comprise entre 0,01 % et 5 % du poids d'eau. Cet extrait de lécithine est un extrait enrichi en phosphatidylcholine.

La phase huileuse est composée principalement d'hydrocarbures paraffiniques huileux et d'un extrait de lécithine liposoluble purifiée en proportion comprise entre 0,01 % et 5 % du poids d'hydrocarbures.

La proportion de la phase huileuse peut varier entre 5 % et 90 % du poids total de l'émulsion.

Avantageusement, les produits selon l'invention contiennent, en outre, une poudre minérale inerte, telle que par exemple du kaolin, du talc ou du carbonate de calcium en proportion comprise entre 0,1 % et 10 % du poids total qui est mise en suspension dans la phase aqueuse.

Selon un mode de réalisation préférentiel, les produits selon l'invention contiennent, en outre, des mono-diglycérides ayant une température de fusion supérieure à 50°C, en proportion comprise entre 0,05 % et 10 % du poids total, qui sont fondus et additionnés à la phase huileuse.

Le terme hydrocarbures paraffiniques est utilisé dans les ouvrages scientifiques pour désigner tous les hydrocarbures saturés de formule $C^n H^{2n+2}$, c'est-à-dire les alcanes.

Il est précisé que ce terme est utilisé dans la présente demande pour désigner des hydrocarbures saturés comportant dans leur molécule, un nombre de carbones élevé, qui se présentent à la température ambiante soit à l'état huileux, soit à l'état solide ou cireux, comme par exemple la vaseline ou l'huile de paraffine ou encore la paraffine proprement dite, qui est un solide qui fond à une température comprise entre 43°C et 65°C. Dans ce dernier cas, la paraffine est fondue avant d'être mélangée à un extrait de lécithine liposoluble, puis elle est mélangée à la phase aqueuse pour former une émulsion à une température où elle est évidemment liquide.

La présence d'un extrait de lécithine liposoluble dans la phase aqueuse permet d'obtenir des émulsions d'eau et d'huile très stables, en utilisant comme agent émulseur, uniquement des extraits de lécithine qui sont des produits d'origine naturelle non toxiques.

On rappelle que les lécithines sont des lipides phosphorés qui se trouvent dans divers tissus végétaux ou animaux.

On trouve dans le commerce de la lécithine extraite notamment du jaune d'oeuf, du lait, de la moelle osseuse, des haricots, du soja etc...

La lécithine joue un rôle important dans les phénomènes de perméabilité des membranes cellulaires et, à ce titre, dans les mécanismes du métabolisme cellulaire.

On trouve dans le commerce des extraits de lécithine qui sont enrichis en phosphatidylcholine et qui sont dispersibles dans l'eau. Ces extraits sont vendus par exemple sous les dénominations commerciales "EPIKURON 145V" ou "EMULPUR N". On trouve également dans le commerce des extraits de lécithine liposolubles purifiés qui sont vendus par exemple sous la dénomination commerciale "EMULFLUID E".

La présente invention permet d'obtenir plusieurs gammes de produits, notamment des produits diététiques ou laxatifs se présentant sous la forme d'une pâte tartinable et des produits cosmétiques se présentant sous la forme de crèmes, de gels ou de laits, applicables sur la peau, pour des soins cutanés.

Les produits diététiques selon l'invention ont l'apparence et la consistance d'un beurre ou d'une margarine, dans lesquels les graisses assimilables par l'organisme sont remplacées en totalité ou en grande partie par une émulsion d'eau et d'hydrocarbures paraffiniques huileux, qui ne sont pas assimilables, de sorte que ces produits ont un pouvoir calorifique très faible.

Si la teneur en hydrocarbures est inférieure à 45 %, on obtient des pâtes à tartiner diététiques dotées de bonnes propriétés olfacto-gustatives et d'une bonne onctuosité, auxquelles on peut ajouter divers arômes tels que banane, noisette, chocolat, fruits pour les pâtes sucrées ou bien saumon fumé, anchois, herbes de Provence etc..., pour des compositions salées.

Lorsque la teneur de l'émulsion en hydrocarbures paraffiniques est comprise entre 45 % et 90 %, on obtient des pâtes tartinables ayant l'aspect d'une margarine dotée de propriétés laxatives qui permettent donc de fournir des produits laxatifs doux sous une nouvelle présentation, plus facile à absorber par certains patients qui sont rebutés par les laxatifs huileux tels que l'huile de paraffine.

Les produits tartinables selon l'invention présentent l'avantage de ne contenir aucune substance tensio-active synthétique, qui serait susceptible de rendre l'huile de paraffine assimilable au niveau du tube digestif. Ils se présentent sous la forme de pâtes tartinables stables, qui ne laissent pas exsuder d'eau, qui s'étalent facilement sur une tartine, qui ne fondent pas à la température ambiante et qui développent dans la bouche des propriétés olfacto-gustatives très satisfaisantes.

On décrit ci-après, à titre d'illustration, quelques exemples non limitatifs de préparations de plusieurs produits tartinables selon l'invention.

EXEMPLE NO. 1 : Préparation d'une pâte à tartiner diététique :

Dans un réacteur double enveloppe en acier inoxydable de 150 litres, équipé d'une prise de vide et d'un agitateur, on prépare d'abord, une phase huileuse.

On introduit dans le réacteur :
– 35 kg d'huile de paraffine qualité codex à haute viscosité ;
– 5 kg de paraffine solide ayant une température de fusion supérieure à 45°C ;
– 0,3 kg de mono-diglycérides solides ayant une température de fusion supérieure à 50°C ;
– 0,5 kg d'extrait de lécithine purifiée du commerce qui est partiellement hydrolysée et qui est titrée en phosphatides et en lysophospholipides liposolubles.

On fait circuler de l'eau chaude entre les deux parois de la double enveloppe pour porter la température à environ 70° à 80°C, jusqu'à ce que les produits solides soient fondus. On homogénéise par agitation à basse vitesse, de l'ordre de 200 T/Minute. On interrompt la circulation d'eau chaude et on laisse revenir la température aux environs de 55° à 60°C. On ajoute un agent colorant permettant d'obtenir une pâte de couleur jaune rappelant la couleur du beurre. Par exemple, on ajoute environ 1 gramme de béta-carotène à 30 %. On ajoute également 100 grammes d'arôme de beurre.

Par ailleurs, on prépare dans une cuve chauffante une phase aqueuse. On verse dans la cuve :
– 50,5 litres d'eau purifiée ;
– 0,5 kg d'extrait de lécithine hydrodispersible, enrichie en phosphatidylcholine que l'on trouve dans le commerce ;
– 0,1 kg d'un agent conservateur, par exemple de benzoate de sodium ;
– 2 kg d'une poudre minérale inerte, par exemple de la poudre de kaolin purifié.

On agite le mélange pour obtenir une suspension homogène et on chauffe à une température de l'ordre de 55° à 60°C.

Un fois la phase aqueuse ainsi préparée, on l'envoie progressivement au moyen d'une pompe dans le réacteur contenant la phase huileuse maintenue sous agitation lente à une température de l'ordre de 55° à 60°C.

Lorsque toute la phase liquide a été transvasée dans le réacteur, on met en route une pompe à vide qui fait baisser la pression jusqu'à une valeur de l'ordre de 6500 Pascal (65 mbars) et on agite fortement à 500 tours/minute pendant 10 minutes environ pour former une émulsion d'eau et d'huile.

Après quoi, on fait baisser la température aux environs de 40°C, de sorte que la paraffine et les mono-diglycérides se figent.

L'émulsion ainsi obtenue est affinée par passage dans un homogénéiseur de type moulin colloïdal, puis on l'extrude à travers une filière sous une pression de 50 à 60 bars. Le produit obtenu est une pâte qui peut être conditionnée en barquettes, puis refroidie. On peut également refroidir la pâte à la sortie de la filière et la découper en pains ou en plaques que l'on conditionne comme du beurre.

EXEMPLE NO. 2 :

On prépare comme précédemment une phase huileuse en plaçant dans un réacteur :
– 36,6 kg d'huile de paraffine qualité codex à basse viscosité ;
– 2,5 Kg d'un hydrocarbure paraffinique solide ayant une température de fusion supérieure à 45°C ;
– 0,4 kg de mono-diglycérides solides ayant une température de fusion supérieure à 50°C ;
– 0,4 kg d'extrait de lécithine purifiée partiellement hydrolysée titrée en lysophospholipides qui est soluble dans les hydrocarbures ;
– 1 g de béta-carotène à 30 % (colorant) ;
– 125 g d'arôme, par exemple arôme chocolat ou noisette.

La préparation de la phase aqueuse est la même que dans l'exemple N0.1 et les étapes du procédé de préparation de l'émulsion sont inchangées.

On obtient ainsi une pâte molle, plus fluide que la précédente, qui peut être consommée comme dessert diététique.

La différence essentielle avec l'exemple NO. 1, réside dans le fait que la quantité de paraffine solide est divisée par deux ce qui explique la consistance plus molle de la pâte.

EXEMPLE NO. 3 : Préparation d'un "beurre" diététique allégé en matières grasses

On prépare une phase huileuse en plaçant dans un réacteur :
– 25 kg d'huile de paraffine qualité codex haute viscosité ;
– 20 kg de beurre ou 15 kg d'huile de beurre ou d'huile végétale ;
– 0,5 kg de mono-diglycérides solides ayant une température de fusion supérieure à 50°C ;
– 3,5 kg de paraffine solide ayant une température de fusion supérieure à 45°C ;
– 0,35 kg d'extrait de lécithine purifiée, partiellement hydrolysée, titrée en lysophospholipides ;
– 100 g d'un arôme par exemple un arôme d'anchois.

La phase aqueuse est la même que dans les deux exemples précédents. Elle est additionnée de sel.

On obtient ainsi un produit diététique qui contient seulement 16 % de matières grasses assimilables et qui se présente sous la forme d'une pâte à tartiner, qui peut être utilisée par exemple pour garnir des toasts.

Dans les exemples qui précèdent, la phase aqueuse contient 100 g d'un agent conservateur qui est du benzoate de sodium. Celui-ci peut être remplacé par de l'acide sorbique, de l'acide salicylique ou par du sorbate de sodium ou de potassium, associé à de l'acide citrique ou ascorbique agissant comme adjuvants de goût et agents anti-oxydants.

Dans la préparation de la phase huileuse pour l'obtention d'un beurre allégé selon l'exemple n° 3, on peut remplacer les 20 kg de beurre par 16 kg d'huile de poisson désodorisée, par exemple de l'huile de flétan, de morue, de saumon etc..., qui présentent un intérêt diététique en raison de leur teneur titrée en acides gras insaturés tel que acide gamma linolénique ou acide linoléique.

L'huile utilisée est sélectionnée en fonction de son traitement désodorisant et de la présence d'agents stabilisants, en particulier anti-oxydants du type vitamine E.

EXEMPLE NO. 4 : Préparation d'une pâte à tartiner laxative :

On prépare la phase huileuse en plaçant dans un réacteur :
 – 65 Kg d'huile de paraffine qualité codex à haute viscosité ;
 – 6 kg de paraffine solide ayant une température de fusion supérieure à 45°C ;
 – 0,5 kg de mono-diglycérides solides ayant une température de fusion supérieure à 50°C ;
 – 0,6 kg d'extrait de lécithine purifiée partiellement hydrolysée titrée en lysophospholipides ;
 – 1 g de béta carotène à 30 % pour obtenir une coloration rappelant celle du beurre ;
 – 150 g d'une composition aromatique.

Les étapes de préparation de la phase aqueuse et du procédé de préparation de l'émulsion sont les mêmes que dans l'exemple n° 1.

On obtient finalement une pâte qui peut être consommée à la cuillère comme un dessert, ou étalée sur une tartine et qui présente des propriétés laxatives dues à sa teneur élevée en huile de paraffine.

Les exemples qui précèdent montrent que les procédés selon l'invention permettent d'obtenir de nouvelles compositions diététiques ou laxatives, composées d'une émulsion d'une phase aqueuse dans une phase huileuse qui est composée principalement d'hydrocarbures paraffiniques huileux ou solides, à la température ambiante, utilisés seuls ou associés à des graisses animales ou végétales. La présence dans ces produits d'un extrait de lécithine liposoluble et d'un extrait hydrodispersible de lécithine enrichi en phosphatidylcholine, permet d'obtenir des émulsions très stables, ne contenant aucun agent tensio-actif de synthèse. Les compositions obtenues sont des produits pâteux utilisables en diététique comme substituts du beurre, comme pâtes tartinables sucrées ou salées ou comme crèmes consommables à la cuillère.

Les procédés selon l'invention d'obtention d'émulsions d'eau dans une phase huileuse riche en hydrocarbures paraffiniques huileux ou solides à la température ambiante, permettent également de préparer des produits cosmétiques applicables sur la peau, dans lesquels les agents tensio-actifs de synthèse sont remplacés par des extraits de lécithine.

Les étapes essentielles des procédés de fabrication restent les mêmes que celles des exemples n° 1 à n° 4, qui précèdent. Seules les compositions changent.

EXEMPLE NO. 5 : Préparation d'une crème de soins

Pour obtenir par exemple 100 Kg de crème, on prépare une phase huileuse en plaçant dans un réacteur :
 – 15 kg de vaseline qui est un mélange d'hydrocarbures paraffiniques ;
 – 15 kg d'huile de paraffine ;
 – 0,5 kg d'extrait de lécithine liposoluble purifiée titrée en lysophospholipides ;
 – 0,75 kg de mono-diglycérides solides ayant une température de fusion supérieure à 50°C ;
 – 3 kg d'une huile végétale, par exemple de l'huile de carthame.

Par ailleurs, on prépare une phase aqueuse contenant :
 – 58 kg d'eau purifiée ;
 – 0,5 kg d'extrait de lécithine hydrodispersible riche en phosphatidylcholine ;
 – 3 kg de poudre minérale inerte par exemple de kaolin ;
 – 5 kg de sorbitol ou de glycérine ;
 – 0,075 kg d'un agent conservateur tel que acide solicylique, sorbique ou benzoïque ;
 – quelques grammes d'un parfum.

Eventuellement, on peut ajouter dans la phase huileuse des pigments en suspension dans le cas de crèmes solaires ou des agents tels que des agents hydratants, acidifiants, régénérateurs, protecteurs, filtrants, selon l'usage auquel la crème est destinée, celle-ci pouvant être une crème de nuit ou de jour, une crème fluide corporelle, une crème pour les mains, une crème capillaire, une crème teintée ou solaire, etc...

Les produits obtenus ont la consistance d'une crème plus ou moins fluide ou pâteuse, facile à étaler sur la peau.

Il est précisé que les quantités indiquées dans l'exemple n° 5 correspondent seulement à un exemple non limitatif.

De façon plus générale, les proportions en poids de chaque produit, ramenées au poids total, peuvent varier entre les limites suivantes dans une crème de soins :

- vaseline entre 0 % et 45 %, de préférence entre 12 % et 25 % ;
- huile de paraffine : entre 2,5 % et 45 %, de préférence entre 12 % et 25 % ;
- extrait de lécithine liposoluble : entre 0,05 % et 10 %, de préférence entre 0,1 % et 3 % ;
- mono-diglycérides : entre 0,05 % et 10 %, de préférence entre 0,1 et 3 % ;
- huile végétale : entre 0 et 10 %, de préférence entre 2 et 8 % ;
- extrait de lécithine hydrodispersible : entre 0,05 % et 10 % de préférence entre 0,1 et 3 % ;
- poudre minérale : entre 0,5 % et 10 %, de préférence entre 0,5 et 6 % ;
- eau : quantité suffisante pour atteindre 100 % ;
- sorbitol ou glycérine : entre 0 et 10 % de préférence entre 2,5 et 6 % ;
- agent conservateur : entre 0,01 et 0,5 %, de préférence entre 0,1 et 0,2 %.

EXEMPLE NO. 6 : Préparation de gels-crèmes :

On utilise les produits suivants, dont les proportions sont exprimées en pourcentage du poids total.

Phase huileuse

- vaseline : entre 0 et 20 %, de préférence entre 0 et 6 % par exemple 0 ;
- huile de paraffine : entre 0 et 20 %, de préférence entre 2 et 15 %, par exemple 11 % ;
- huile végétale : entre 0 et 20 %, de préférence entre 2 et 10 %, par exemple 8 % ;
- extrait de lécithine liposoluble : entre 0,05 % et 20 %, de préférence entre 0,2 et 10 %, par exemple 0,5 % ;
- mono-diglycérides : entre 0 et 6 %, de préférence entre 0 et 0,6 %, par exemple 0.

Phase aqueuse

- sorbitol ou glycérine : entre 0 et 10 %, de préférence entre 0 et 5 %, par exemple 0 ;
- poudre minérale inerte : entre 0 et 10 %, de préférence entre 0,5 et 6 %, par exemple 0,5 % ;
- extrait de lécithine hydrodispersible : entre 0,05 et 10 %, de préférence entre 0,1 et 3 %, par exemple 1,5 % ;
- agent gélifiant : entre 0 et 1 %, de préférence 0,2 et 1 %, par exemple 0,7 % (par exemple polycarboxypolyvinyle dit "Carbopol") ;
- alcool éthylique : entre 1 et 60 %, de préférence entre 1 et 50 %, par exemple 10 %.

En sus des constituants essentiels ci-dessus, on peut ajouter quelques gouttes d'un parfum et quelques gouttes de triéthanol amine pour régler la viscosité du produit.

Les produits selon l'exemple n° 6, peuvent être utilisés par exemple comme gels ou crèmes pour les soins du visage ou du corps, comme crèmes solaires, comme excipients pour des préparations magistrales.

Ils diffèrent des crèmes selon l'exemple n° 5 par le fait qu'ils contiennent de l'alcool, une moindre quantité de phase huileuse et un agent gélifiant qui permet d'obtenir une consistance de gel.

EXEMPLE NO. 7 : Préparation d'un lait crème démaquillant

Les proportions sont exprimées en pourcentage du poids total.

Phase huileuse

- vaseline : entre 0,5 % et 10 %, de préférence entre 0,5 et 2 % par exemple 0,5 % ;
- huile de paraffine : entre 0,5 et 10 %, de préférence entre 0,5 et 2 %, par exemple 0,5 % ;
- extrait de lécithine liposoluble : entre 0,01 et 2 %, de préférence entre 0,01 et 0,5 %, par exemple 0,05 % ;
- huile végétale : entre 0 et 10 %, de préférence entre 0 et 5 %, par exemple 0 .
- mono-diglycérides : entre 1 et 6 %, de préférence entre 2,5 et 5 %, par exemple 2,5 % ;
- agent conservateur : entre 0 et 0,5 %, de préférence entre 0,1 et 0,2 %, par exemple 0,1 % (acide salicylique ou benzoïque) ;

Phase aqueuse

- sorbitol ou glycérine : entre 0 et 10 %, de préférence entre 2,5 et 6 %, par exemple 2,5%.
- eau : quantité nécessaire pour atteindre 100 % ;
- extrait de lécithine : entre 0,05 et 10 %, de préférence entre 1 et 3 %, par exemple 1,75 % ;
- poudre minérale inerte : entre 0,5 et 10 %, de préférence entre 0,5 et 6 %, par exemple 2,5 % (par exemple kaolin) ;
- agent gélifiant : entre 0 et 0,5 %, de préférence entre 0,1 et 0,25 %, par exemple 0,15 % (par exemple polycarboxy polyvinyle).

On peut ajouter quelques gouttes de parfum et quelques gouttes de triéthanol amine pour régler la viscosité.

EXEMPLE NO. 8 : Préparation d'une base excipient à faible teneur en eau

Les proportions sont exprimées en pourcentage du poids total.

Phase huileuse

– vaseline : entre 20 et 90 %, de préférence entre 50 et 70 % par exemple 68 % ;
– huile de paraffine : entre 2 et 20 %, de préférence entre 5 et 10 %, par exemple 7 %.
– extrait de lécithine liposoluble : entre 0,05 et 10 %, de préférence entre 0,2 et 2 %, par exemple 1 % ;
– huile végétale : entre 0 et 10 %, de préférence entre 0,5 et 2 %, par exemple 1,7 % ;
– mono-diglycérides : entre 0,05 et 10 %, de préférence entre 0,5 et 2 %, par exemple 1,7 % ;
– agent conservateur : entre 0 et 0,5 %, de préférence entre 0,1 et 0,2 %, par exemple 0,1 % ;

Phase aqueuse

– sorbitol ou glycérine : entre 0 et 20 %, de préférence entre 2 et 10 %, par exemple 10 % ;
– eau : quantité nécessaire pour atteindre 100 %, par exemple 7 % ;
– extrait de lécithine : entre 0,05 et 10 %, de préférence entre 0,1 et 3 %, par exemple 0,9 % ;
– poudre minérale inerte : entre 0,5 et 10 %, de préférence entre 0,5 et 6 %, par exemple 2 %.

On peut ajouter quelques gouttes de parfum et entre 0 et 2 % de vitamine E.

Cette base excipient, qui a une très faible teneur en eau, peut absorber son poids d'eau au moment de l'utilisation.

Elle peut servir d'excipient auquel on peut incorporer une vitamine A acide, du sulfate de zinc, de l'érythromycine.

Les exemples 5 à 8 décrivent divers produits cosmétiques destinés notamment aux soins de la peau, qui se présentent tous sous la forme d'une émulsion d'une phase aqueuse dans une phase huileuse, composée essentiellement d'hydrocarbures paraffiniques huileux, tels que huile de paraffine ou vaseline et qui contiennent à la fois un extrait de lécithine soluble dans la phase huileuse et un extrait de lécithine à haute teneur en phosphatidylcholine qui est dispersible dans la phase aqueuse.

Les hydrocarbures paraffiniques remplissent la fonction de corps gras utilisés habituellement dans les produits dermatologiques pour conférer à ceux-ci leur onctuosité et permettre de les étaler sur la peau sous forme d'une mince pellicule.

Les extraits de lécithine remplacent les agents tensio-actifs qui sont habituellement utilisés pour stabiliser les émulsions.

La présence d'extraits de lécithine dans ces produits dermatologiques accroît leur efficacité. En effet, la lécithine appliquée sur le peau joue un rôle de surface très important. Elle a des propriétés hydratantes, reconstituantes et protectrices. Elle augmente la souplesse et l'élasticité de la peau et la respiration cutanée. Par ailleurs elle protège la peau contre les effets délipidants des agents tensio-actifs.

Appliquée aux cheveux, la lécithine produit des effets adoucissants, elle donne du volume, elle améliore la coiffabilité, elle réduit les charges électrostatiques.

La lécithine a déjà été utilisée comme adjuvant dans certains produits cosmétiques. Toutefois son emploi a été limité au rôle d'adjuvant, du fait qu'elle est insoluble dans l'eau et, à ce jour, elle a été utilisée uniquement comme composant accessoire de la phase huileuse de certains produits cosmétiques ou en association avec des agents tensio-actifs synthétiques.

Les compositions selon l'invention permettent d'incorporer des proportions plus importantes d'extraits de lécithine liposolubles ou hydrodispersibles, sans addition d'aucun agent tensio-actif synthétique.

Les produits cosmétiques selon l'invention sont préparés à l'aide d'agents émulgateurs naturels et d'hydorcarbures paraffiniques raffinés, qui ne présentent aucune agressivité, ni toxicité. Ce sont de véritables formulations physiologiques présentant des caractères communs avec les structures naturelles et les composants des membranes cellulaires.

Ils présentent une bio-compatibilité exceptionnelle qui se manifeste par l'absence totale de toute irritation cutanée ou allergique telles que rougeurs, échauffements, vasodilatation, desquamation, démangeaisons etc...

Cet avantage est particulièrement sensible pour les produits qui doivent rester au contact de la peau ou des muqueuses pendant une durée prolongée.

Des essais réalisés avec des compositions cosmétiques selon l'invention ont démontré une tolérance exceptionnelle, même dans le cas d'applications sur une peau abrasée ou sur des plaies. Dans ce dernier cas, les patients ont apprécié la sensation de fraîcheur, l'effet apaisant et l'accélération de la cicatrisation des plaies.

Tous les produits décrits dans les exemples 1 à 8 présentent le caractère commun d'être constitués par une émulsion d'une phase acqueuse et d'une phase huileuse avec une proportion de phase huileuse comprise entre 5 % et 90 %, laquelle phase huileuse est composée en totalité ou en majorité d'hydrocarbures paraffiniques qui sont huileux ou solides à la température ambiante, qui sont évidemment liquides au moment où l'on prépare l'émulsion et qui sont additionnés d'un extrait de lécithine liposoluble utilisé dans des proportions comprises entre 0,01 % et 5 % du poids d'hydrocarbures et laquelle phase aqueuse contient un extrait de lécithine hydro-dispersible dans des proportions comprises entre 0,01 % et 5 % du poids d'eau, lequel extrait de lécithine que l'on trouve dans le commerce est un extrait ayant une teneur élevée en phosphatidylcholine qui est dispersible dans l'eau.

En plus des constituants essentiels énumérés ci-dessus, les produits selon l'invention contiennent avantageusement une poudre minérale inerte, par exemple du kaolin, du talc ou du carbonate de calcium qui est mise en suspension dans la phase aqueuse dans des proportions comprises entre 0,1 et 10 % du poids total de l'émulsion. Cette poudre minérale a un effet stabilisateur de l'émulsion.

De plus, les produits selon l'invention peuvent également contenir avantageusement des mono-diglycérides qui ont un point de fusion supérieur à 50°C dans des proportions comprises entre 0,05 % et 10 % du poids total de l'émulsion. Ces mono-diglycérides ont pour fonction de conférer aux produits une consistance pâteuse à la température ambiante.

Avantageusement, les produits selon l'invention peuvent contenir de petites quantités d'agents de conservation tels que de l'acide benzoïque, salicylique ou sorbique, ou contenir également une petite quantité d'huile végétale ou de poisson ou du beurre, des parfums, des vitamines, des colorants etc...

## Revendications

**Revendications pour les Etats contractants suivants : DE, GB, IT, NL, SE, LI, CH, BE, AT, LU**

1. Produits composés d'une émulsion d'une phase aqueuse et d'une phase huileuse dans la proportion de 5 % à 90 % du poids total, contenant des hydrocarbures paraffiniques huileux ou solides à la température ambiante, additionnés de lécithine liposoluble en proportion comprise entre 0,01 % et 5 % du poids d'hydrocarbures paraffiniques, CARACTERISES EN CE QUE la phase aqueuse contient un extrait de lécithine hydrodispersible, enrichi en phosphatidylcholine en proportion comprise entre 0,01 % et 5 % du poids d'eau.

2. Produits selon la revendication 1, CARACTERISES EN CE QU'ils contiennent, en outre, une poudre minérale inerte en proportion comprise entre 0,1 % et 10 % du poids total.

3. Produits selon l'une quelconque des revendications 1 et 2, CARACTERISES EN CE QU'ils contiennent, en outre, des mono-diglycérides ayant un point de fusion supérieur à 50°C, en proportion comprise entre 0,05 % et 10 % du poids total.

4. Produits pâteux tartinables selon l'une quelconque des revendications 1 à 3, CARACTERISES EN CE QU'ils contiennent une proportion d'hydrocarbures paraffiniques ayant un point de fusion supérieur à 50°C comprise entre 0,2 % et 10 % du poids total.

5. Produits diététiques tartinables selon la revendication 3, CARACTERISES EN CE QU'ils contiennent une proportion totale d'hydrocarbures paraffiniques huileux ou solides à la température ambiante comprise entre 10 % et 45 % du poids total.

6. Produits laxatifs tartinables selon la revendication 3, CARACTERISES EN CE QU'ils contiennent une proportion totale d'hydrocarbures paraffiniques huileux ou solides à la température ambiante comprise entre 45 % et 90 % du poids total.

7. Produits cosmétiques selon la revendication 3, utilisables comme crèmes ou gels, applicables sur la peau, CARACTERISES EN CE QU'ils contiennent les proportions suivantes de produits exprimées en pourcentage du poids total :
   – vaseline : entre 0 et 45 %, de préférence entre 12 et 25 % ;
   – huile de paraffine : entre 2,5 et 45 %, de préférence entre 12 et 25 % ;
   – extrait de lécithine liposoluble : entre 0,05 % et 10 %, de préférence entre 0, 1 et 3 % ;
   – mono-diglycérides : entre 0,05 % et 10 %, de préférence entre 0,1 et 3 % ;
   – huile végétale : entre 0 et 10 %, de préférence entre 2 et 8 % ;
   – extrait de lécithine hydrodispersible : entre 0,05 % et 10 %, de préférence entre 0,1 et 3 % ;
   – poudre minérale inerte : entre 0,5 % et 10 %, de préférence entre 0,5 et 6 % ;
   – sorbitol ou glycérine : entre 0 et 10 %, de préférence entre 2,5 et 6 % ;
   – agent conservateur : entre 0,01 et 0,5 %, de préférence entre 0,1 et 0,2 % ;
   – eau : quantité nécessaire pour atteindre 100 %.

8. Produits cosmétiques selon la revendication 3, utilisables comme gel ou crèmes applicables sur la peau, CARACTERISES EN CE QU'ils contiennent les proportions de produits suivantes, exprimées en pourcentage du poids total :
   – vaseline : entre 0 et 20 %, de préférence entre 0 et 6 % ;
   – huile de paraffine : entre 0 et 20 %, de préférence entre 2 et 15 % ;

– extrait de lécithine liposoluble : entre 0,05 et 20 %, de préférence entre 0,2 et 10 % ;

– mono-diglycérides : entre 0 et 6 %, de préférence entre 0 et 0,6 % ;

– huile végétale : entre 0 et 20 %, de préférence entre 2 et 10 % ;

– extrait de lécithine hydrodispersible : entre 0,05 et 10 %, de préférence entre 0,1 et 3 % ;

– poudre minérale inerte : entre 0 et 10 %, de préférence entre 0,5 et 6 % ;

– sorbitol ou glycérine : entre 0 et 10 %, de préférence entre 0 et 5 % ;

– alcool éthylique : entre 1 et 60 %, de préférence entre 1 et 50 % ;

– agent gélifiant : entre 0 et 1 %, de préférence entre 0,2 et 1 % ;

– agent conservateur : entre 0 et 0,5 %, de préférence entre 0,1 et 0,2 % ;

– eau : quantité nécessaire pour atteindre 100 %.

9. Produits cosmétiques selon la revendication 3, utilisables comme laits ou crèmes démaquillants, CARACTERISES EN CE QU'ils contiennent les proportions de produits suivantes exprimées en pourcentage du poids total :

– vaseline : entre 0,5 et 10 %, de préférence entre 0,5 et 2 % ;

– huile de paraffine : entre 0,5 et 10 %, de préférence entre 0,5 et 2% ;

– extrait de lécithine liposoluble : entre 0,01 et 2 %, de préférence entre 0,01 et 0,5 % ;

– mono-diglycérides : entre 1 et 6 %, de préférence entre 2,5 et 5 % ;

– huile végétale : entre 0 et 10 %, de préférence entre 0 et 5 % ;

– extrait de lécithine hydrodispersible : entre 0,05 et 10 % de préférence entre 1 et 3 % ;

– poudre minérale inerte : entre 0,5 et 10 %, de préférence entre 0,5 et 6 % ;

– sorbitol ou glycérine : entre 0 et 10 %, de préférence entre 2,5 et 6 % ;

– agent gélifiant : entre 0 et 0,5 %, de préférence entre 0, 1 et 0,25 % ;

– agent conservateur : entre 0 et 0,5 %, de préférence entre 0,1 et 0,2 % ;

– eau : quantité nécessaire pour atteindre 100 %.

10. Produits cosmétiques selon la revendication 3, utilisables comme base ou excipient à faible teneur en eau, CARACTERISES EN CE QU'ils contiennent les proportions de produits suivantes, exprimées en pourcentage du poids total :

– vaseline : entre 20 et 90 %, de préférence entre 50 et 70 %;

– huile de paraffine : entre 2 et 20 %, de préférence entre 5 et 10 % ;

– extrait de lécithine liposoluble : entre 0,05 et 10 %, de préférence entre 0,2 et 2 % ;

– mono-diglycérides : entre 0,05 et 10 %, de préférence entre 0,5 et 2 % ;

– huile végétale : entre 0 et 10 %, de préférence entre 0,5 et 2 % ;

– sorbitol ou glycérine : entre 0 et 20 %, de préférence entre 2 et 10 % ;

– extrait de lécithine hydrodispersible : entre 0,05 et 10 %, de préférence entre 0,1 et 3 % ;

– poudre minérale inerte : entre 0,5 et 10 %, de préférence entre 0,5 et 6 % ;

– agent conservateur : entre 0 et 0,5 %, de préférence entre 0,1 et 0,2 % ;

– eau : quantité nécessaire pour atteindre 100 %.

11. Procédé de fabrication d'un produit selon la revendication 1, CARACTERISE PAR la suite d'opérations suivantes :

– on prépare une phase huileuse en plaçant dans un réacteur lesdits hydrocarbures paraffiniques liquides et/ou solides, à la température ambiante et ledit extrait de lécithine purifiée liposoluble, on chauffe à une température de l'ordre de 70°C, on agite, on ramène à une température de l'ordre de 55 à 60°C ;

– on prépare dans un récipient une phase liquide contenant de l'eau et un extrait de lécithine enrichi en phosphatidylcholine, qui est mis en suspension dans l'eau, on agite et on chauffe à une température de l'ordre de 55 à 60°C ;

– on transvase progressivement la phase aqueuse dans le réacteur contenant la phase huileuse maintenue à environ 55°C en agitant celle-ci ;

– on met le réacteur en dépression en maintenant dans celui-ci une pression de l'ordre de 6500 Pascal ;

– on agite fortement pendant une dizaine de minutes pour former une émulsion ;

– on abaisse la température aux environs de 40°C ;

– et on affine l'émulsion par passage dans un moulin colloïdal.

12. Procédé selon la revendication 11 pour fabriquer un produit selon la revendication 3, CARACTERISE EN CE QUE l'on ajoute, en outre, dans le réacteur contenant la phase huileuse, des mono-diglycérides ayant une température de fusion supérieure à 50°C et on ajoute une poudre minérale en suspension dans la phase aqueuse, telle que du kaolin ou talc ou du carbonate de calcium.

**Revendications pour les Etats contractants suivants : GR, ES**

1. Procédé de fabrication de produits composés d'une émulsion d'une phase aqueuse et d'une phase huileuse dans la proportion de 5 % à 90 % du poids total, contenant des hydrocarbures paraffiniques huileux ou solides à la température ambiante, additionnés de lécithine liposoluble en proportion comprise entre 0,01 % et 5 % du poids d'hydrocarbures paraffiniques, CARACTERISE PAR la suite d'opérations suivantes :

   – on prépare une phase huileuse en plaçant dans un réacteur lesdits hydrocarbures paraffiniques liquides et/ou solides, à la température ambiante et ledit extrait de lécitihine purifiée liposoluble, on chauffe à une température de l'ordre de 70°C, on agite, on ramène à une température de l'ordre de 55 à 60°C ;

   – on prépare dans un récipient une phase liquide contenant de l'eau et un extrait de lécithine enrichi en phosphatidylcholine, qui est mis en suspension dans l'eau, on agite et on chauffe à une température de l'ordre de 55 à 60°C ;

   – on transvase progressivement la phase aqueuse dans le réacteur contenant la phase huileuse maintenue à environ 55°C en agitant celle-ci ;

   – on met le réacteur en dépression en maintenant dans celui-ci une pression de l'ordre de 6 500 Pascal ;

   – on agite fortement pendant une dizaine de minutes pour former une émulsion ;

   – on abaisse la température aux environs de 40°C ;

   – et on affine l'émulsion par passage dans un moulin colloïdal.

2. Procédé selon la Revendication 1, CARACTERISE EN CE QUE l'on ajoute, en outre, dans le réacteur contenant la phase huileuse, des mono-diglycérides ayant une température de fusion supérieure à 50°C et on ajoute une poudre minérale en suspension dans la phase aqueuse, telle que du kaolin ou talc ou du carbonate de calcium.

**Patentansprüche**

**Patentansprüche für folgende Vertragsstaaten : DE, GB, IT, NL, SE, LI, CH, BE, AT, LU**

1. Produkte, die eine Emulsion aus einer wäßrigen Phase und einer öligen Phase in einer Menge von 5 bis 90%, bezogen auf das Gesamtgewicht, darstellen, die ölige oder bei Raumtemperatur feste Paraffinkohlenwasserstoffe enthält, die mit fettlöslichem Lecithin in einer Menge zwischen 0,01 und 5%, bezogen auf das Gewicht der Paraffinkohlenwasserstoffe, versetzt sind, dadurch **gekennzeichnet**, daß die wäßrige Phase einen Extrakt an wasserdispergierbarem Lecithin enthält, der mit Phosphatidylcholin in einer Menge zwischen 0,01 und 5%, bezogen auf das Gewicht des Wassers, angereichert ist.

2. Produkte nach Anspruch 1, dadurch **gekennzeichnet**, daß sie außerdem ein inertes Mineralpulver in einer Menge zwischen 0,1 und 10%, bezogen auf das Gesamtgewicht, enthalten.

3. Produkte nach einem der Ansprüche 1 und 2, dadurch **gekennzeichnet**, daß sie außerdem Mono-Diglyceride mit einem Schmelzpunkt von über 50°C in einer Menge zwischen 0,05 und 10%, bezogen auf das Gesamtgewicht, enthalten.

4. Streichfähige pastöse Produkte nach einem der Ansprüche 1 bis 3, dadurch **gekennzeichnet**, daß sie Paraffinkohlenwasserstoffe mit einem Schmelzpunkt von über 50°C in einer Menge zwischen 0,2 und 10%, bezogen auf das Gesamtgewicht, enthalten.

5. Streichfähige diätetische Produkte nach Anspruch 3, dadurch **gekennzeichnet**, daß sie eine Gesamtmenge an öligen oder bei Raumtemperatur festen Paraffinkohlenwasserstoffen zwischen 10 und 45%, bezogen auf das Gesamtgewicht, enthalten.

6. Abführende streichfähige Produkte nach Anspruch 3, dadurch gekennzeichnet, daß sie eine Gesamtmenge an öligen oder bei Raumtemperatur festen Paraffinkohlenwasserstoffen :wischen 45 und 90%, bezogen auf das Gesamtgewicht, enthalten.

7. Kosmetische Produkte nach Ansprüch 3, die als auf die Haut auftragbare Cremes oder Gele verwendbar sind, dadurch **gekennzeichnet**, daß sie die nachfolgenden Mengen an Komponenten, ausgedrückt in Prozentanteilen, bezogen auf das Gesamtgewicht, enthalten:

   – Vaseline: 0 - 45%, vorzugsweise 12 - 25%;

   – Paraffinöl: 2,5 - 45%, vorzugsweise 12 - 25%;

   – Extrakt von fettlöslichem Lecithin: 0,05 - 10%, vorzugsweise 0,1 - 3%;

   – Mono-Diglyceride: 0,05 - 10%, vorzugsweise 0,1 - 3%;

   – Pflanzenöl: 0 - 10%, vorzugsweise 2 - 8%;

   – Extrakt von wasserdispergierbarem Lecithin: 0,05 - 10%, vorzugsweise 0,1 - 3%;
– inertes Mineralpulver: 0,5 - 10%, vorzugsweise 0,5 - 6%;
– Sorbit oder Glycerin: 0 - 10%, vorzugsweise 2,5 - 6%;
– Konservierungsmittel: 0,01 - 0,5%, vorzugsweise 0,1 - 0,2%;
– Wasser: erforderliche Menge zur Erreichung von 100%.

8. Kosmetische Produkte nach Anspruch 3, die als auf die Haut auftragbare Cremes oder Gele verwendbar sind, dadurch **gekennzeichnet**, daß sie die nachfolgenden Mengen an Komponenten, ausgedrückt in Prozentanteilen, bezogen auf das Gesamtgewicht, enthalten:
   – Vaseline: 0 - 20%, vorzugsweise 0 - 6%;
   – Paraffinöl: 0 - 20%, vorzugsweise 2 - 15%;
   – Extrakt von fettlöslichem Lecithin: 0,05% - 20%, vorzugsweise 0,2 - 10%;
   – Mono-Diglyceride: 0 - 6%, vorzugsweise 0 - 0,6%;
   – Pflanzenöl: 0 - 20%, vorzugsweise 2 - 10%;
   – Extrakt von wasserdispergierbarem Lecithin: 0,05-10%, vorzugsweise 0,1 - 3%;
   – inertes Mineralpulver: 0 - 10%, vorzugsweise 0,5 - 6%;
   – Sorbit oder Glycerin: 0 - 10%, vorzugsweise 0 - 5%;
   – Ethylalkohol: 1 - 60%, vorzugsweise 1 - 50%;
   – Gelierungsmittel: 0 - 1%, vorzugsweise 0,2 - 1%;
   – Konservierungsmittel: 0 - 0,5%, voerzugsweise 0,1 - 0,2%;
   – Wasser: erforderliche Menge zur Erreichung von 100%.

9. Kosmetische Produkte nach Anspruch 3, die als auf die Haut auftragbare Reinigungsmilch oder -cremes verwendbar sind, dadurch **gekennzeichnet**, daß sie die nachfolgenden Mengen an Komponenten, ausgedrückt in Prozentanteilen, bezogen auf das Gesamtgewicht, enthalten:
   – Vaseline: 0,5 - 10%, vorzugsweise 0,5 - 2%;
   – Paraffinöl: 0,5 - 10%, vorzugsweise 0,5 - 2%;
   – Extrakt von fettlöslichem Lecithin: 0,01% - 2%, vorzugsweise 0,01 - 0,5%;
   – Mono-Diglyceride: 1 - 6%, vorzugsweise 2,5 - 5%;
   – Pflanzenöl: 0 - 10%, vorzugsweise 0 - 5%;
   – Extrakt von wasserdispergierbarem Lecithin: 0,05 -10%, vorzugsweise 1 - 3%;
   – inertes Mineralpulver: 0,5 - 10%, vorzugsweise 0,5 -6%;
   – Sorbit oder Glycerin: 0 - 10%, vorzugsweise 2,5 - 6%;

– Gelierungsmittel: 0 - 0,5%, vorzugsweise 0,1 - 0,25%;
– Konservierungsmittel: 0 - 0,5%, vorzugsweise 0,1 -0,2%;
– Wasser: erforderliche Menge zur Erreichung von 100%.

10. Kosmetische Produkte nach Anspruch 3, die als Basis bzw. Träger mit geringem Wassergehalt verwendbar sind, dadurch **gekennzeichnet**, daß sie die nachfolgenden Mengen an Komponenten, ausgedrückt in Prozentanteilen, bezogen auf das Gesamtgewicht, enthalten:
   – Vaseline: 20 - 90%, vorzugsweise 50 - 70%;
   – Paraffinöl: 2 - 20%, vorzugsweise 5 - 10%;
   – Extrakt von fettlöslichem Lecithin: 0,05% - 10%, vorzugsweise 0,2 - 2%;
   – Mono-Diglyceride: 0,05 - 10%, vorzugsweise 0,5 - 2%;
   – Pflanzenöl: 0 - 10%, vorzugsweise 0,5 - 2%;
   – Sorbit oder Glycerin: 0 - 20%, vorzugsweise 2 - 10%;
   – Extrakt von wasserdispergierbarem Lecithin: 0,05 - 10%, vorzugsweise 0,1 - 3%;
   – inertes Mineralpulver: 0,5 - 10%, vorzugsweise 0,5 - 6%;
   – Konservierungsmittel: 0 - 0,5%, vorzugsweise 0,1 - 0,2%;
   – Wasser: erforderliche Menge zur Erreichung von 100%.

11. Verfahren zur Herstellung eines Produktes nach Anspruch 1, **gekennzeichnet** durch die nachfolgenden Stufen:
   – Herstellung einer öligen Phase, indem man einen Reaktor mit den genannten flüssigen und/oder festen Paraffinkohlenwasserstoffen bei Raumtemperatur sowie mit dem Extrakt aus gereinigtem fettlöslichem Lecithin beschickt, auf eine Temperatur von 70°C erwärmt, rührt und wieder auf eine Temperatur von 55 bis 60°C abkühlt;
   – Herstellung einer Flüssigphase, die Wasser und einen mit Phosphatidylcholin angereicherten und in Wasser suspendierten Lecithinextrakt enthält, in einem Gefäß, Rühren und Erwärmen auf eine Temperatur von 55 bis 60°C;
   – kontinuierliche Beschickung des die bei ca. 55°C gehaltene ölige Phase enthaltenden Reaktors mit der wäßrigen Phase, wobei man die ölige Phase rührt;
   – Entspannung des Reaktors auf einen Druck von 6500 Pa;
   – starkes Rühren während ca. 10 min zur Bildung einer Emulsion;
   – Absenken der Temperatur auf ca. 40°C;
   – Homogenisierung der Emulsion durch Passage einer Kolloidmühle.

12. Verfahren nach Anspruch 11 zur Herstellung eines Produktes nach Anspruch 3, dadurch **gekennzeichnet**, daß man außerdem noch dem die ölige Phase enthaltenden Reaktor Mono-Diglyceride mit einem Schmelzpunkt von über 50°C und ein in der wäßrigen Phase suspendiertes Mineralpulver, wie z.B. Kaolin, Talk oder Calciumcarbonat, zuführt.

**Patentansprüche für folgende Vertragsstaaten : GR, ES**

1. Verfahren zur Herstellung von Produkten, die eine Emulsion aus einer wäßrigen Phase und einer öligen Phase in einer Menge von 5 bis 90%, bezogen auf das Gesamtgewicht, darstellen, die ölige oder bei Raumtemperatur feste Paraffinkohlenwasserstoffe enthält, die mit fettlöslichem Lecithin in einer Menge zwischen 0,01 und 5%, bezogen auf das Gewicht der Paraffinkohlenwasserstoffe, versetzt sind, **gekennzeichnet** durch die nachfolgenden Stufen:
    – Herstellung einer öligen Phase, indem man einen Reaktor mit den genannten flüssigen und/oder festen Paraffinkohlenwasserstoffen bei Raumtemperatur sowie mit dem Extrakt aus gereinigtem fettlöslichem Lecithin beschickt, auf eine Temperatur von 70°C erwärmt, rührt und wieder auf eine Temperatur von 55 bis 60°C abkühlt;
    – Herstellung einer Flüssigphase, die Wasser und einen mit Phosphatidylcholin angereicherten und in Wasser suspendierten Lecithinextrakt enthält, in einem Gefäß, Rühren und Erwärmen auf eine Temperatur von 55 bis 60°C;
    – kontinuierliche Beschickung des die bei ca. 55°C gehaltene ölige Phase enthaltenden Reaktors mit der wäßrigen Phase, wobei man die ölige Phase rührt;
    – Entspannung des Reaktors auf einen Druck von 6500 Pa;
    – starkes Rühren während ca. 10 min zur Bildung einer Emulsion;
    – Absenken der Temperatur auf ca. 40°C;
    – Homogenisierung der Emulsion durch Passage einer Kolloidmühle.

2. Verfahren nach Anspruch 1, dadurch **gekennzeichnet**, daß man außerdem noch dem die ölige Phase enthaltenden Reaktor Mono-Diglyceride mit einem Schmelzpunkt von über 50°C und ein in der wäßrigen Phase suspendiertes Mineralpulver, wie z.B. Kaolin, Talk oder Calciumcarbonat, zuführt.

**Claims**

**Claims for the following Contracting States :
DE, GB, IT, NL, SE, LI, CH, BE, AT, LU**

1. Products composed of an emulsion of an aqueous phase and an oily phase in the proportion of 5% to 90% of the total weight, containing paraffinic hydrocarbons which are oily or solid at ambient temperature, to which liposoluble lecithin is added in a proportion of between 0.01% and 5% of the weight of the paraffinic hydrocarbons, characterised in that the aqueous phase contains an extract of hydrodispersible lecithin, enriched with phosphatidylcholine in a proportion of between 0.01% and 5% of the weight of water.

2. Products according to Claim 1, characterised in that they furthermore contain an inert mineral powder in a proportion of between 0.1% and 10% of the total weight.

3. Products according to one of Claims 1 and 2, characterised in that they moreover contain mono-diglycerides having a melting point higher than 50°C, in a proportion of between 0.05% and 10% of the total weight.

4. Spreadable pasty products according to one of Claims 1 to 3, characterised in that they contain a proportion of paraffinic hydrocarbons having a melting point higher than 50°C of between 0.2% and 10% of the total weight.

5. Spreadable dietetic products according to Claim 3, characterised in that they contain a total proportion of paraffinic hydrocarbons, which are oily or solid at ambient temperature, of between 10% and 45% of the total weight.

6. Spreadable laxative products according to Claim 3, characterised in that they contain a total proportion of paraffinic hydrocarbons, which are oily or solid at ambient temperature, of between 45% and 90% of the total weight.

7. Cosmetic products according to Claim 3, which can be used as creams or gels and can be applied to the skin, characterised in that they contain the following proportions of products expressed as a percentage of the total weight:
    – vaseline: between 0 and 45%, preferably between 12 and 25%;
    – paraffin oil: between 2.5 and 45%, preferably between 12 and 25%;
    – extract of liposoluble lecithin: between 0.05% and 10%, preferably between 0.1 and 3%;

– mono-diglycerides: between 0.05% and 10%, preferably between 0.1 and 3%;
– vegetable oil: between 0 and 10%: preferably between 2 and 8%;
– extract of hydrodispersible lecithin: between 0.05% and 10%, preferably between 0.1 and 3%;
– inert mineral powder: between 0.5% and 10%, preferably between 0.5 and 6%;
– sorbitol or glycerine: between 0 and 10%, preferably between 2.5 and 6%;
– preservative: between 0.01 and 0.5%, preferably between 0.1 and 0.2%;
– water: quantity necessary for reaching 100%.

8. Cosmetic products according to Claim 3, which can be used as a gel or cream to be applied to the skin, characterised in that they contain the proportions of the following products, expressed as a percentage of the total weight:
– vaseline: between 0 and 20%, preferably between 0 and 6%;
– paraffin oil: between 0 and 20%, preferably between 2 and 15%;
– extract of liposoluble lecithin: between 0.05 and 20%, preferably between 0.2 and 10%;
– mono-diglycerides: between 0 and 6%, preferably between 0 and 0.6%;
– vegetable oil: between 0 and 20%, preferably between 2 and 10%;
– extract of hydrodispersible lecithin: between 0.05 and 10%, preferably between 0.1 and 3%;
– inert mineral powder: between 0 and 10%, preferably between 0.5 and 6%;
– sorbitol or glycerine: between 0 and 10%, preferably between 0 and 5%;
– ethyl alcohol: between 1 and 60%, preferably between 1 and 50%;
– gelling agent: between 0 and 1%, preferably between 0.2 and 1%;
– preservative: between 0 and 0.5%, preferably between 0.1 and 0.2%;
– water: quantity necessary for reaching 100%.

9. Cosmetic products according to Claim 3, which can be used as milks or creams for removing make-up, characterised in that they contain the proportions of the following products expressed as a percentage of the total weight:
– vaseline: between 0.5 and 10%, preferably between 0.5 and 2%;
– paraffin oil: between 0.5 and 10%, preferably between 0.5 and 2%;
– extract of liposoluble lecithin: between 0.01 and 2%, preferably between 0.01 and 0.5%;
– mono-diglycerides: between 1 and 6%, preferably between 2.5 and 5%;
– vegetable oil: between 0 and 10%, preferably between 0 and 5%;
– extract of hydrodispersible lecithin: between 0.05 and 10%, preferably between 1 and 3%;
– inert mineral powder: between 0.5 and 10%, preferably between 0.5 and 6%;
– sorbitol or glycerine: between 0 and 10%, preferably between 2.5 and 6%;
– gelling agent: between 0 and 0.5%, preferably between 0.1 and 0.25%;
– preservative: between 0 and 0.5%, preferably between 0.1 and 0.2%;
– water: quantity necessary for reaching 100%.

10. Cosmetic products according to Claim 3, able to be used as a base or excipient having a low water content, characterised in that they contain the proportions of the following products, expressed as a percentage of the total weight:
– vaseline: between 20 and 90%, preferably between 50 and 70%;
– paraffin oil: between 2 and 20%, preferably between 5 and 10%;
– extract of liposoluble lecithin: between 0.05 and 10%, preferably between 0.2 and 2%;
– mono-diglycerides: between 0.05 and 10%, preferably between 0.5 and 2%;
– vegetable oil: between 0 and 10%, preferably between 0.5 and 2%;
– sorbitol or glycerine: between 0 and 20%, preferably between 2 and 10%;
– extract of hydrodispersible lecithin: between 0.05 and 10%, preferably between 0.1 and 3%;
– inert mineral powder: between 0.5 and 10%, preferably between 0.5 and 6%;
– preservative: between 0 and 0.5%, preferably between 0.1 and 0.2%;
– water: necessary quantity to reach 100%.

11. Method of manufacture of a product according to Claim 1, characterised by the succession of the following operations:
– an oily phase is prepared by placing in a reactor said liquid and/or solid paraffinic hydrocarbons, at ambient temperature and said extract of liposoluble purified lecithin, it is heated to a temperature of the order of 70°C, stirred and returned to a temperature of the order of 55 to 60°C;
– in a container one prepares a liquid phase containing water and an extract of lecithin enriched with phosphatidylcholine, which is placed in suspension in water, stirred and heated to a temperature of the order of 55 to 60°C;

– the aqueous phase is decanted progressively into the reactor containing the oily phase kept at a temperature of approximately 55°C with stirring of the latter;

– the reactor is placed under reduced pressure by maintaining a pressure of the order of 6500 Pascals therein;

– vigorous stirring proceeds for about ten minutes in order to form an emulsion;

– the temperature is lowered to approximately 40°C;

– the emulsion is refined by passing through a colloidal mill.

12. Method according to Claim 11 for manufacturing a product according to Claim 3, characterised in that mono-diglycerides having a melting point in excess of 50°C are furthermore added to the reactor containing the oily phase and one adds a mineral powder in suspension in the aqueous phase, such as kaolin or talc or calcium carbonate.

**Claims for the following Contracting States : GR, ES**

1. Method of manufacture of products composed of an emulsion of an aqueous phase and an oily phase in the proportion of 5% to 90% of the total weight, containing paraffinic hydrocarbons which are oily or solid at ambient temperature, to which liposoluble lecithin is added in a proportion of between 0.01% and 5% of the weight of the paraffinic hydrocarbons, characterised by the succession of the following operations:

– an oily phase is prepared by placing in a reactor said liquid and/or solid paraffinic hydrocarbons, at ambient temperature and said extract of liposoluble purified lecithin, it is heated to a temperature of the order of 70°C, stirred and returned to a temperature of the order of 55 to 60°C;

– in a container one prepares a liquid phase containing water and an extract of lecithin enriched with phosphatidylcholine, which is placed in suspension in water, stirred and heated to a temperature of the order of 55 to 60°C;

– the aqueous phase is decanted progressively into the reactor containing the oily phase kept at a temperature of approximately 55°C with stirring of the latter;

– the reactor is placed under reduced pressure by maintaining a pressure of the order of 6,500 Pascals therein;

– vigorous stirring proceeds for about ten minutes in order to form an emulsion;

– the temperature is lowered to approximately 40°C;

– the emulsion is refined by passing through a colloidal mill.

2. Method according to Claim 1, characterised in that monodiglycerides having a melting point in excess of 50°C are furthermore added to the reactor containing the oily phase and one adds a mineral powder in suspension in the aqueous phase, such as kaolin or talc or calcium carbonate.